# FASCICULE DE BREVET EUROPEEN

(11) **EP 2 489 347 B1**
(45) Date de publication et mention de la délivrance du brevet: **01.04.2015**
(21) Numéro de dépôt: 11001416.4
(22) Date de dépôt: 21.02.2011
(51) Int. Cl.: A61K 9/08, A61K 31/05, A61K 9/00

(54) **Procédé de production de formes injectables de phloroglucinol, et les formes injectables ainsi réalisées**
Verfahren zur Herstellung von injizierbaren Zusammensetzungen von Phloroglucinol und die so erhaltenen Zusammensetzungen
Process for the production of injectable preparations of phloroglucinol and the injectable preparations so obtained

(43) Date de publication de la demande: 22.08.2012
(73) Titulaire: Société Delpharm s.a.s., 92130 Issy Les Moulineaux (FR)
(72) Inventeur: Aguettant, Sébastien, 75016 Paris (FR); Ladjimi, Hamed, 91650 Breuillet (FR)
(74) Mandataire: Tripoz, Inès

(56) Documents cités:
- WO-A1-00/71110
- FR-A1- 2 779 061
- GB-A- 904 955
- Anonymous: "SPASFON, SOLUTION INJECTABLE EN AMPOULE", , [Online] 5 juin 2008 (2008-06-05), pages 1-2, XP002592607, therapeutique.info Extrait de l'Internet: URL:http://www.therapeutique.info/rcp.php? code=5076> [extrait le 2010-07-20]
- NICOLAS K KAMBIA ET AL: "Stability and compatibility of the ready-to-use solution of paracetamol admixed with phloroglucinol for intravenous infusion", EJHP SCIENCE, vol. 12, no. 5, 1 janvier 2006 (2006-01-01), pages 91-95, XP002457172, PHARMA PUBLISHING & MEDIA EUROPE, MOL, BE ISSN: 1781-7595
- Anonymous: "RETRAITS DE PRODUITS PHARMACEUTIQUES", , [Online] 25 décembre 2007 (2007-12-25), page 1, XP002592608, SNAPO Extrait de l'Internet: URL:http://www.snapo.org/files/retrait1512 07.pdf> [extrait le 2010-07-20]

## Description

L'invention se situe dans le domaine des nécessités de la vie et plus particulièrement dans le domaine de la pharmacie galénique.

Elle a plus particulièrement pour objet un nouveau procédé de production de formes pharmaceutiques injectables à base d'agent antispasmodique phénolique.

Elle a précisément pour objet un nouveau procédé de production de formes injectables, stables et non oxydables à base de phloroglucinol et de son ester triméthylique en solution ou en dispersion dans un véhicule aqueux non toxique, pharmaceutiquement acceptable.

Ce procédé est caractérisé en ce que l'on procède à l'élimination de l'oxygène dissout dans le véhicule aqueux par barbotage d'un gaz inerte insoluble dans l'eau et par l'ajustement judicieux du pH de la solution.

Il était connu que les solutions injectables de phloroglucinol comme nombre de solutions injectables de substances phénoliques comme le paracétamol ou la dobutamine ont tendance à s'oxyder en présence de quantités minimes d'oxygène et que la désoxygénation du milieu constitue un des moyens utilisables pour éviter que ces solutions ne se colorent soit au cours de la stérilisation à la chaleur soit au cours de la conservation. Un des moyens utilisés a été d'introduire dans le milieu un capteur d'oxygène ou un agent d'inertage faisant intervenir la vapeur blanche d'eau bi-distillée ou un agent anti oxydant come le metabisulfite de sodium.

La multiplicité des méthodes décrites pour stabiliser de telles solutions amène à penser qu'il y en a peu qui soient pleinement satisfaisantes.

En outre l'inertage par gaz inerte n'est pas sans poser des problèmes car les gaz inertes comme le xénon, le néon ou même l'argon contiennent aussi des quantités décelables d'oxygène qui sont susceptibles d'amorcer une auto-oxydation.

La présente invention vise à résoudre ces difficultés et à apporter une solution plus satisfaisante à la production de solutions injectables oxydables.

L'invention concerne spécifiquement un procédé de production de solutions injectables de phloroglucinol sous forme dihydraté et de triméthylphloroglucinol, dans l'eau qui a l'avantage d'être plus stable que la solution injectable du médicament princeps, le SPASFON®.

La stabilité de cette solution est conditionnée par l'ajustement judicieux du pH et l'élimination de l'oxygène dissout.

L'élimination de l'oxygène dissout s'effectue commodément par barbotage d'un gaz inerte insoluble dans l'eau et, de préférence par barbotage de l'azote.

Cependant le gaz utilisé pour le barbotage de la solution en vue de chasser l'oxygène peut être un gaz rare comme l'argon ou le xénon mais le gaz préféré est l'azote.

Le barbotage de l'azote se fait tout au long de la fabrication afin d'assurer une teneur en oxygène inférieure à 0,1 ppm dans la cuve de fabrication et dans la cuve de stockage. Le barbotage d'azote se fait également dans la cuve de transfert ainsi que dans les ampoules avant et après répartition.

L'ajustement du pH s'effectue par addition ménagée d'un acide minéral fort comme par exemple l'acide chlorydrique.

Cette préparation injectable doit pouvoir être stérilisée par la chaleur.

Pour l'administration parentérale et intraveineuse, les solutions doivent être ajustées à l'isotonie. L'isotonie de la préparation peut être obtenue par ajout d'une quantité judicieusement calculée d'un agent isotonisant et en particulier de chlorure de sodium.

La composition pourra être stérilisée par traitement thermique, par exemple à 121°C pendant 20 minutes ou par filtration stérilisante sur membrane Amicon de porosité inférieure à 0,22µm.

### EXEMPLE 1

Choix du pH de la solution

Des solutions de phloroglucinol/triméthylphloroglucinol ont été fabriquées dans une gamme de pH allant de 3,0 à 4,0 par addition calculée d'acide chlorhydrique.

La composition des solutions est présentée ci-après.

| **Compositions de solutions** | | | | | | |
|---|---|---|---|---|---|---|
| **Phloroglucinol dihydrate** | x | x | x | x | x | x |
| **Triméthylphloroqlucinol** | x | x | x | x | x | x |
| **Chlorure de sodium** | x | x | x | x | x | x |
| **pH** | 3,0 | 3,2 | 3,4 | 3,6 | 3,8 | 4,0 |

Chacune de ces solutions est conditionnée dans des flacons et inertée par de l'azote durant 30 sec. Les solutions sont stérilisées par la chaleur durant 20 min à 121 °C puis conservées à +40°C/HR75%

### Stabilité à T= 1 mois à +40°C/HR75%

| | **Produits de dégradation (exprimé en % phloroglucinol)** | | | | | | | |
|---|---|---|---|---|---|---|---|---|
| ***Trr** | **2,7** | **3,0** | **3,2** | **3,4** | **3,5** | **3,6** | **3,8** | **Total** |
| Solution pH3.0 | 0,000 | 0,015 | 0,001 | 0,004 | 0,001 | 0,000 | 0,013 | 0,034 |
| Solution pH3.2 | 0,000 | 0,013 | 0,002 | 0,004 | 0,000 | 0,000 | 0,013 | 0,032 |
| Solution pH3.4 | 0,000 | 0,026 | 0,004 | 0,003 | 0,003 | 0,000 | 0,012 | 0,048 |
| Solution pH3.6 | 0,000 | 0,033 | 0,006 | 0,003 | 0,003 | 0,000 | 0,008 | 0,053 |
| Solution pH3.8 | 0,000 | 0,035 | 0,008 | 0,003 | 0,001 | 0, 000 | 0,012 | 0,059 |
| Solution pH4.0 | 0,000 | 0,044 | 0,009 | 0,003 | 0,003 | 0,000 | 0,006 | 0,065 |
| | | | | | | | | |
| **SPASFON^{®}, lot R8168 (pH4.0), ampoule non conservé à +40°C** | 0,422 | 0,017 | 0,001 | 0,000 | 0,000 | 0,003 | 0,000 | 0,443 |

| | | | | | | | | |
|---|---|---|---|---|---|---|---|---|
| *^{*}Trr : Temps de rétention relatif par rapport pic de phloroglucinol* | | | | | | | | |

### Interprétation des résultats :

Les résultats des analyses montrent :
   - l'absence du pic de Trr 2,7 observé avec la solution de SPASFON®,
   - la présence d'un pic de Trr 3,0. Sa surface reste relativement stable en pH 3,0 et 3,2 puis augmente proportionnellement avec le pH.
   - la présence d'un pic Trr 3,8 de faible surface.

Il convient de noter que la solution à pH 4,0 se colore en jaune après 1 mois de conservation à +40°C.

### Stabilité à T= 3 mois à +40°C/HR75%

| | **Produits de dégradation (exprimé en % phloroglucinol)** | | | | | | | | | |
|---|---|---|---|---|---|---|---|---|---|---|
| ***Trr** | **2,2** | **2,7** | **3.0** | **3,05** | **3,1** | **3,2** | **3,4** | **3,5** | **3,6** | **Total** |
| Solution pH3.0 | 0,003 | 0,008 | 0,102 | 0,000 | 0,002 | 0,002 | 0,006 | 0,005 | 0,009 | **0,137** |
| Solution pH3.2 | 0,003 | 0,005 | 0,113 | 0,001 | 0,003 | 0,001 | 0,005 | 0,004 . | 0,009 | **0,144** |
| Solution pH3.4 | 0,004 | 0,008 | 0,163 | 0,001 | 0,007 | 0,005 | 0,005 | 0,003 | 0,008 | **0,204** |
| Solution pH3.6 | 0,004 | 0,006 | 0,150 | 0,002 | 0,008 | 0,005 | 0,004 | 0,004 | 0,007 | **0,190** |
| Solution pH3.8 | 0,005 | 0,006 | 0,158 | 0,004 | 0,012 | 0,009 | 0,003 | 0,004 | 0,007 | **0,207** |
| Solution pH4.0 | 0,007 | 0,009 | 0,220 | 0,007 | 0,020 | 0,017 | 0,004 | 0,004 | 0,005 | **0,295** |
| | | | | | | | | | | |
| **SPASFON^{®}, lot R8168 (pH4.0), ampoule non conservé à +40°C** | 0.000 | **0,422** | **0,017** | 0,000 | 0,001 | 0,001 | 0,000 | **0,000** | **0,003** | **0,444** |

| | | | | | | | | | | |
|---|---|---|---|---|---|---|---|---|---|---|
| *^{*} Trr : Temps de rétention relatif par rapport pic de phloroglucinol* | | | | | | | | | | |

### Coloration des solutions

| **Nature des solutions** | **Aspect des solutions** |
|---|---|
| Solution pH3.0 | Incolore |
| Solution pH3.2 | Incolore |
| Solution pH3.4 | Jaune à T= 3 mois |
| Solution pH3.6 | Jaune à T = 2 mois |
| Solution pH3.8 | Jaune à T = 45 jours |
| Solution pH4.0 | Jaune à T = 1 mois |

### Interprétation des résultats

Les résultats des analysent montrent :
- la présence à l'état de trace du pic de Trr 2,7 observé avec la solution de SPASFON®,
- la présence d'un pic Trr 3,0, dont la surface est relativement stable entre pH 3,0 et 3,2 puis augmente significativement à partir de pH 3,4.

Les solutions ajustées à pH 3,0 et 3,2 restent incolores et présentent les plus faibles pourcentages en produits de dégradation.

| ***Stabilité à T= 6 mois à +40°C*/*HR75%*** | | | | | | | | | | | | | |
|---|---|---|---|---|---|---|---|---|---|---|---|---|---|
| | **Produits de dégradation (exprimé en % phloroglucinol)** | | | | | | | | | | | | |
| **^{*}Trr** | **0,9** | **2,2** | **2,7** | **2,8** | **3,0** | **3,05** | **3,1** | **3,2** | **3,3** | **3,4** | **3,5** | **3,6** | **Total** |
| **Solution pH3.0** | 0,005 | 0,007 | 0,013 | 0,006 | 0,300 | - | - | 0,002 | - | 0,015 | 0,015 | 0,011 | 0,374 |
| **Solution pH3.2** | 0,006 | 0,008 | 0,012 | 0,006 | 0,382 | - | - | 0,004 | 0,009 | 0,012 | 0,014 | - | 0,453 |
| **Solution pH3.4** | 0,006 | 0,008 | 0,011 | 0,008 | 0,372 | - | 0, 009 | 0,005 | 0,016 | 0,012 | 0,011 | - | 0,458 |
| **Solution pH3.6** | 0,006 | 0,008 | 0,007 | 0,006 | 0,420 | - | 0,013 | 0,009 | 0,025 | 0,012 | 0,011 | - | 0,517 |
| **Solution pH3.8** | 0,006 | 0,011 | 0,008 | 0,012 | 0,464 | 0.006 | 0,017 | 0, 014 | 0, 039 | 0,011 | 0,010 | - | 0,598 |
| **Solution pH4.0** | 0,007 | 0.010 | 0,012 | 0,013 | 0,551 | 0,014 | 0,024 | 0,026 | 0,065 | 0,010 | 0,007 | - | 0,739 |
| | | | | | | | | | | | | | |
| **SPASFON^{®}, lot R8168 (pH4.0), ampoule non conservé à +40°C** | 0,000 | 0,000 | 0,422 | 0,000 | 0,017 | 0,000 | 0,001 | 0,001 | - | - | 0,000 | 0,003 | 0,444 |

| | | | | | | | | | | | | | |
|---|---|---|---|---|---|---|---|---|---|---|---|---|---|
| ** Trr : Temps de rétention relatif par rapport pic de phloroglucinol* | | | | | | | | | | | | | |

### Coloration des solutions

| **Nature des solutions** | **Aspect des solutions** |
|---|---|
| Solution pH3.0 | Jaune à T=6 mois |
| Solution pH3.2 | Jaune à T=4 mois |
| Solution pH3.4 | Jaune à T= 3 mois |
| Solution pH3.6 | Jaune à T = 2 mois |
| Solution pH3.8 | Jaune à T = 45 jours |
| Solution pH4.0 | Jaune à T = 1 mois |

### Interprétation des résultats

Les résultats des analyses montrent :
- la présence à l'état de trace du pic de Trr 2,7 observé avec la solution de SPASFON®,
- la présence d'un pic de Trr 3,0. Sa surface augmente proportionnellement au pH.
- La solution ajustée à pH 3,0 devient jaune. Les autres solutions sont fortement colorées en jaune.
- Le plus faible pourcentage en produits de dégradation est observé avec la solution à pH 3.0. Le pourcentage en impuretés totales augmente proportionnellement au pH.

### Conclusion

La solution de Phloroglucinol/triméthylphloroglucinol ajustée à pH égal à 3,0 présente la meilleure stabilité. La solution de SPASFON® présente à T=0 un pic de Trr 2.7 de surface assez importante. Ce pic est présent à l'état de trace dans la solution Phloroglucinol/triméthylphloroglucinol selon l'invention et n'évolue pas en cours de conservation de la solution à +40°C. Cependant, un pic de Trr 3.0 apparaît avec la solution de Phloroglucinol/triméthylphloroglucinol. Son intensité varie proportionnellement au pH. Il pourrait être à l'origine de l'apparition de la coloration jaune de la solution.

### EXEMPLE II

Stabilité d'une solution de Phloroglucinol/triméthylphloroglucinol fabriquée à l'échelle industrielle *versus* le produit princeps : SPASFON^{®}.

Effet de la teneur résiduelle en oxygène dans la solution.

Composition de la solution de Phloroglucinol/triméthylphloroglucinol injectable :

| **Désignation** | **Composition unitaire** |
|---|---|
| **Phloroglucinol dihydrate** | 40 mg |
| **Triméthylphloroglucinol** | 0,040 mg |
| **Chlorure de sodium** | 0,028 g |
| **HCl** | qsp pH 3,0 |
| **EPPI** | 4 ml |

L'effet de l'inertage par l'azote des solutions de Phloroglucinol/triméthylphloroglucinol en cours de répartition dans les ampoules a été effectuée à travers les essais effectués ci-après.
- **Essai 1** : sans préazotage et sans postazotage des ampoules durant la répartition
- **Essai 2** : avec préazotage et sans postazotage des ampoules durant la répartition
- **Essai 3** : avec préazotage et avec postazotage des ampoules durant la répartition

Les analyses des ampoules stérilisées conservées durant 3 mois à +40°C/HR75% versus la spécialité SPASFON^{®}, du lot 8264, sont présentées dans le tableau ci-après.

| **ESSAl 1** | | | |
|---|---|---|---|
| **Nature des contrôles** | **Spécifications** | **T0** | **T = 3 mois à +40°C/HR75%** |
| Aspect | Solution limpide, incolore à légèrement jaunâtre ≤ JBS | Conforme | Légèrement jaune <JB5 |
| pH | 2,9 à 3,3 | 3,09 | 3,14 |
| Dosage du phloroglucinol dihydrate | 38,0 à 42,0 mg/ampoule | 40,4 (101,0%) | 40,2 (100,5%) |
| Dosage du triméthylphtoroglucinol | 38,0 à 42,0 µg/ampoule | 40,0 (100,0%) | 39,8 (99,5%) |
| Produits de dégradation | - Impureté individuelle inconnue (II) ≤ 0,2% | II: 0,009 | II: 0,005-0,006-0,005-0,095 |
| | - Impuretés totales (IT) Spécification provisoire ≤ 0,5% | IT : 0,009 | IT : 0,111 |

| **ESSAI 2** | | | |
|---|---|---|---|
| **Nature des contrôles** | **Specifications** | **T0** | **T = 3 mois à +40°C/HR75%** |
| Aspect | Solution limpide, incolore à légèrement jaunâtre ≤ JBS | Conforme | Légèrement jaune <JB5 |
| pH | 2,9 à 3,3 | 3,09 | 3,12 |
| Dosage du phloroglucinol dihydrate | 38.0 à 42,0 mg/ampoule | 40,2 (100,5%) | 40,3 (100,8%) |
| Dosage du triméthylphloroglucinol | 38,0 à 42,0 µg/ampoule | 40,0 (100,0%) | 40,4 (101,0%) |
| Produits de dégradation | - Impureté individuelle inconnue (II) ≤ 0,2% | II : 0,010 | II : 0,110 |
| | - Impuretés totales (IT) Spécification provisoire ≤ 0,5% | IT : 0,010 | IT : 0,110 |

| **ESSAI 3** | | | |
|---|---|---|---|
| **Nature des contrôles** | **Spécifications** | **T0** | **T = 3 mois à +40°C/HR75%** |
| Aspect | Solution limpide, incolore à légèrement jaunâtre ≤ JB5 | Conforme | Conforme |
| pH | 2,9 à 3,3 | 3,08 | 3,16 |
| Dosage de l'API200a | 38,0 à 42.0 mg/ampoule | 40,1 (100,3%) | 40,3 (100,8%) |
| Dosage de l'API200b | 38,0 à 42,0 µg/ampoule | 39,6 (99,0%) | 40,2 (100,5%) |
| Produits de dégradation | Impureté individuelle inconnue (II) ≤ 0,2% | II : 0,007-0,007 | II : 0,043 |
| | - Impuretés totales (IT) Spécification provisoire ≤ 0,5% | IT : 0,014 | IT: 0,043 |

| **SPASFON^{®}, lot 8264** | | | |
|---|---|---|---|
| **Nature des contrôles** | **Spécifications** | **T0** | **T = 3 mois à +40ºC/HR75%** |
| Aspect | Solution limpide, incolore à légèrement jaunâtre ≤ JB5 | Conforme | Légèrement jaune <JB5 |
| pH | 2,9 à 3,3 | 3,89 | 4,29 |
| Teneur en oxygène dans les ampoules | ≤ 0,5 ppm | x | x |
| Dosage du phloroglucinol dihydrate | 38,0 à 42,0 mg/ampoule | 39,9 (99,8%) | 39, 9 (99,8%) |
| Dosage du triméthylphloroglucinol | 38,0 à 42,0 µg/ampoule | 40,3 (100,8%) | 40,0 (100,0%) |
| Produits de dégradation | - Impureté individuelle inconnue (II) ≤ 0,2% | II : 0,007-0,453-0,021 | II : 0,008- 0,464-0,136-0,006-0,015 |
| | - Impuretés totales (IT) Spécification provisoire ≤ 0,5% | IT : 0,481 | IT : 0,629 |

La comparaison des résultats obtenus avec ceux des essais 1, 2 et 3 montrent l'importance de la teneur en oxygène résiduel dans la stabilité de la solution de Phloroglucinol/triméthylphloroglucinol injectable.

La comparaison des résultats de l'essai 3 et ceux obtenus avec la spécialité SPASFON^{®} confirment l'importance que joue également le pH dans la stabilité de la solution.

## Revendications

1. Procédé de production de formes injectables stables et non oxydables à base de phloroglucinol et de son éther triméthylique en solution ou en dispersion dans un véhicule aqueux dans lequel on procède à l'élimination de l'oxygène dissout tout au long de la fabrication et dans la cuve de stockage, et dans lequel on ajuste le pH de la solution à une valeur variant de 3,0 à 4,0, par addition d'un acide minéral fort.

2. Procédé selon la revendication 1 dans lequel la teneur en oxygène au cours de la fabrication est inférieure à 0,1 ppm.

3. Procédé selon la revendication 1 et la revendication 2 dans lequel l'élimination de l'oxygène s'effectue par barbotage d'un gaz inerte insoluble dans l'eau.

4. Procédé selon l'une des revendications précédentes dans lequel le gaz inerte insoluble dans l'eau est l'azote ou un gaz rare.

5. Procédé selon l'une des revendications précédentes dans lequel l'acide minéral fort est l'acide chlorhydrique.

6. Procédé selon l'une des revendications précédentes dans lequel l'ajustement du pH de la solution s'effectue entre pH 3,0 et 3,2.

7. Procédé selon l'une des revendications précédentes dans lequel les solutions sont ajustées à l'isotonie par addition d'un agent isotonisant comme le chlorure de sodium.

8. Procédé selon l'une des revendications précédentes dans lequel les solutions sont stérilisées par traitement thermique ou par filtration stérilisante.

9. Les solutions aqueuses, stables, injectables de phloroglucinol et de son éther triméthylique chaque fois qu'elles sont obtenues par le procédé de la revendication 1.

## Patentansprüche

1. Verfahren zum Herstellen stabiler und nicht-oxidierbarer injizierbarer Formen auf Basis von in einem wässrigen Träger gelöstem oder dispergiertem Phloroglucinol und seinem Trimethylether, wobei das Verfahren umfasst das Entfernen des gelösten Sauerstoffes stets während der Herstellung und in dem Vorratsgefäß, und das Einstellen des pH-Wertes der Lösung auf einen Wert zwischen 3,0 und 4,0 durch Zugabe einer starken anorganischen Säure.

2. Verfahren nach Anspruch 1, wobei der Sauerstoffgehalt während der Herstellung geringer als 0,1 ppm ist.

3. Verfahren nach einem der Ansprüche 1 und 2, wobei das Entfernen des Sauerstoffes erfolgt durch Einleiten eines in Wasser unlöslichen Inertgases.

4. Verfahren nach einem der vorangehenden Ansprüche, wobei das in Wasser unlösliche Inertgas Stickstoff oder ein Edelgas ist.

5. Verfahren nach einem der vorangehenden Ansprüche, wobei die starke anorganische Säure Salzsäure ist.

6. Verfahren nach einem der vorangehenden Ansprüche, wobei das Einstellen des pH-Wertes der Lösung zwischen pH 3,0 und 3,2 erfolgt.

7. Verfahren nach einem der vorangehenden Ansprüche, wobei die Lösungen isotonisch eingestellt werden durch Zugabe eines isotonisierenden Mittels wie Natriumchlorid.

8. Verfahren nach einem der vorangehenden Ansprüche, wobei die Lösungen sterilisiert werden durch thermische Behandlung oder Sterilfiltration.

9. Wässrige, stabile, injizierbare Lösungen von Phloroglucinol und seinem Trimethylether, wie sie gemäß dem Verfahren nach Anspruch 1 erhalten werden.

## Claims

1. Process for the production of stable and non-oxidizable injectable forms based on phloroglucinol and on its trimethyl ether in solution or in dispersion in an aqueous vehicle, in which the dissolved oxygen is removed throughout the manufacture and in the storage vessel and in which the pH of the solution is adjusted to a value varying from 3.0 to 4.0 by addition of a strong inorganic acid.

2. Process according to Claim 1, in which the oxygen content during the manufacture is less than 0.1 ppm.

3. Process according to Claim 1 and Claim 2, in which the removal of the oxygen is carried out by sparging with an inert water-insoluble gas.

4. Process according to one of the preceding claims, in which the inert water-insoluble gas is nitrogen or a rare gas.

5. Process according to one of the preceding claims, in which the strong inorganic acid is hydrochloric acid.

6. Process according to one of the preceding claims, in which the adjustment of the pH of the solution is carried out between pH 3.0 and 3.2.

7. Process according to one of the preceding claims, in which the solutions are adjusted to isotonicity by addition of an isotonicity agent, such as sodium chloride.

8. Process according to one of the preceding claims, in which the solutions are sterilized by heat treatment or by sterilizing filtration.

9. Injectable stable aqueous solutions of phloroglucinol and of its trimethyl ether each time that they are obtained by the process of Claim 1.
